Europäisches Patentamt

(19) European Patent Office

Office européen des brevets

(11) Publication number: **0 308 199**
A1

# (12) EUROPEAN PATENT APPLICATION

(21) Application number: **88308498.0**

(22) Date of filing: **14.09.88**

(51) Int. Cl.⁴: **C 12 N 15/00**
C 12 N 1/20, C 12 P 21/02
//(C12N1/20,C12R1:19)

(30) Priority: **16.06.88 US 207690    18.09.87 US 98359**

(43) Date of publication of application:
**22.03.89 Bulletin   89/12**

(84) Designated Contracting States:
**AT BE CH DE ES FR GB GR IT LI LU NL SE**

(71) Applicant: **Mycogen Corporation**
**5451 Oberlin Drive**
**San Diego, CA 92121  (US)**

(72) Inventor: **Gilroy, Thomas E.**
**10050 Osgood Way**
**San Diego California 92126  (US)**

(74) Representative: **Perry, Robert Edward et al**
**GILL JENNINGS & EVERY 53-64 Chancery Lane**
**London WC2A 1HN  (GB)**

The applicant has filed a statement in accordance with Rule 28 (4) EPC (issue of a sample only to an expert). Accession number of the deposit: NRRL B - 18252.

(54) **Bacillus thuringiensis toxin.**

(57) A novel B.t.i. toxin gene toxic to dipteran insects has been cloned. The DNA encoding the B.t.i. toxin can be used to transform various prokaryotic and eukaryotic microbes to express the B.t.i. toxin. These recombinant microbes can be used to control dipteran insects, e.g. mosquitoes, in various environments.

EP 0 308 199 A1

Description

## BACILLUS THURINGIENSIS TOXIN

### Field of the Invention

This invention relates to a Bacillus Thuringiensis clone toxic to dipteran insects.

### Background of the Invention

The spore-forming microorganism Bacillus thuringiensis (B.t.) produces the best-known insect toxin. The toxin is a protein, synthesized by the B.t. sporulating cell. The toxin, upon being ingested in its crystalline form by susceptible insect larvae, is transformed into biologically-active moieties by the insert gut juice proteases. The primary target is insect cells of the gut epithelium, which are rapidly destroyed. Experience has shown that the activity of the B.t. toxin is so high that only nanogram amounts are required to kill susceptible insect larvae.

The reported activity spectrum of B.t. covers insect species within the order Lepidoptera, which constitute a major insect problem in agriculture and forestry. The activity spectrum also includes the insect order Diptera which comprises some species of mosquitoes and blackflies. See Couch (1980) and Beegle (1978), Developments in Industrial Microbiology, respectively 22:61-67 and 20:97-104.

### Summary of the Invention

This invention provides a novel toxin gene toxic to dipteran insects, e.g. mosquitoes. This gene was derived from a Bacillus thuringiensis var. israelensis (B.t.i.) microbe identified as strain HD567.

More specifically, the subject invention concerns DNA encoding a ca. 130 kilodalton (kd) protein having activity against dipteran insects, vectors and hosts, and the novel B.t.i. toxin itself. The invention also concerns formulation and methods for use of the B.t.i. toxin to control dipteran insects.

A DNA nucleotide sequence encoding the novel B.t.i. toxin, and the amino-acid sequence of the novel B.t.i. toxin, are tabulated in claim 1. The invention includes all nucleotide sequences which, by virtue of the redundancy of the genetic code, encode the same amino-acid sequence. It has been shown that proteins of identified structure and function may be constructed by changing the amino-acid sequence if such changes do not alter the protein secondary structure (Kaiser, E.T. and Kezdy, F.J. [1984] Science 223:249-255). Thus, the present invention includes mutants of the amino-acid sequence tabulated in claim 1 which do not alter the protein secondary structure, or, if the structure is altered, the biological activity is retained to some degree.

### Disclosure of the Invention

The B.t.i. isolate used as the source of the toxin gene of the subject invention is identified as B.t.i. strain HD567. This culture is available from Howard T. Dulmage, Cotton Insects Laboratory, United States Department of Agriculture Laboratory, P.O. Box 1033, Brownsville, TX 78520, USA. This culture can be grown using standard techniques and media well known in the art. The B.t.i. cells can be harvested by standard techniques, e.g. centrifugation. The recovered B.t.i. cells are then converted to protoplasts by standard methods. Thereafter cellular DNA is extracted by well-known procedures. The resulting purified DNA is the starting material for digestion with suitable restriction endonucleases.

A gene bank of B.t.i. can then be constructed. In the subject invention, the purified B.t.i. DNA, obtained as described above, was digested with the restriction endonucleases EcoRI and HindIII. The EcoRI and HindIII fragment, after purification, was ligated into the well-known and available plasmid pUC19 which had been cleaved with EcoRI and HindIII.

Once the gene bank of B.t.i. DNA was constructed, it then became necessary to construct a DNA probe to screen the gene bank. The probe was labelled and hybridized by procedures known in the art. The net result was the detection of positive clones, i.e., those that hybridized to the constructed probe.

The recombinant plasmids isolated from representative positive clones were found to have an ca. 3.5 kilobase (kb) EcoRI-HindIII DNA fragment. This ca. 3.5 kb DNA fragment was sequenced by the standard Sanger dideoxy chain termination method.

The toxin gene of the subject invention can be introduced into a wide variety of microbial hosts. Expression of the toxin gene results, directly or indirectly, in the intracellular production and maintenance of the pesticide.

With suitable hosts, e.g., Pseudomonas, the microbes can be applied to the situs of dipteran insects where they will proliferate and be ingested by the insects. The result is a control of the unwanted insects. Alternatively, the microbe hosting the toxin gene can be treated under conditions that prolong the activity of the toxin produced in the cell. The treated cell then can be applied to the environment of target pest(s). The resulting product retains the toxicity of the B.t.i. toxin.

Where the B.t.i. toxin gene is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, e.g., genera Pseudomonas, Erwinia, Serratia, Klebsiella, Xanthomonas, Streptomyces, Rhizobium, Rhodopseudomonas, Methylophilius, Agrobacterium, Acetobacter, Lactobacillus, Arthrobacter, Azotobacter, Leuconostoc, and Alcaligenes; fungi, particularly yeast, e.g., genera Saccharomyces, Cryptococcus, Kluyveromyces, Sporobolomyces, Rhodotorula, and Aureobasidium. Of particular interest are such phytosphere bacterial species as Pseudomonas syringae, Pseudomonas fluorescens, Serratia marcescens, Acetobacter xylinum, Agrobacterium tumefaciens, Rhodopseudomonas spheroides, Xanthomonas campestris, Rhizobium melioti, Alcaligenes entrophus, and Azotobacter vinlandii; and phytosphere yeast species such as Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae, and Aureobasidium pollulans. Of particular interest are the pigmented microorganisms.

A wide variety of ways are available for introducing the B.t.i. gene expressing the toxin into the microorganism host under conditions which allow for stable maintenance and expression of the gene. One can provide for DNA constructs which include the transcriptional and translational regulatory signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will only occur after release into the environment. This can be achieved with operators or a region binding to an activator or enhancers, which are capable of induction upon a change in the physical or chemical environment of the microorganisms. For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger, particularly by using an active promoter, as well as by employing sequences, which enhance the stability of the messenger RNA. The initiation and translational termination region will involve stop codon(s), a terminator region, and optionally, a polyadenylation signal.

In the direction of transcription, namely in the 5' to 3' direction of the coding or sense sequence, the construct will involve the transcriptional regulatory region, if any, and the promoter, where the regulatory region may be either 5' or 3' of the promoter, the ribosomal binding site, the initiation codon, the structural gene having an open reading frame in phase with the initiation codon, the stop codon(s), the polyadenylation signal sequence, if any, and the terminator region. This sequence as a double strand may be used by itself for transformation of a microorganism host, but will usually be included with a DNA sequence involving a marker, where the second DNA sequence may be joined to the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototrophy to an auxotrophic host, or the like. Preferably, complementation is employed, so that the modified host may not only be selected, but may also be competitive in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin-producing host, so that it may effectively compete with the wild-type microorganisms and stably occupy a niche in the environment.

Where no functional replication system is present, the construct will also include a sequence of at least 50 basepairs (bp), preferably at least about 100 bp, and usually not more than about 1000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that a toxin gene is lost, the resulting organism will be likely to also lose the complementing gene and/or the gene providing for the competitive advantage, so that it will be unable to compete in the environment with the gene retaining the intact construct.

A large number of transcriptional regulatory regions are available from a wide variety of microorganism hosts, such as bacteria, bacteriophage, cyanobacteria, algae, fungi, and the like. Various transcriptional regulatory regions include the regions associated with the trp gene, lac gene, gal gene, the lambda left and right promoters, the Tac promoter, the naturally-occurring promoters associated with the toxin gene, where functional in the host. See for example, U.S. Patent Nos. 4,332,898, 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host. The replication system may be derived from the chromosome, an episomal element normally present in the host or a different host, or a replication system from a virus which is stable in the host. A large number of plasmids are available, such as pBR322, pACYC184, RSF1010, pRO1614, and the like. See for example, Olson et al., (1982) J. Bacteriol. 150:6069, and Bagdasarian et al., (1981) Gene 16:237, and U.S. Patent Nos. 4,356,270, 4,362,817, and 4,371,625.

The B.t.i. gene can be introduced between the transcriptional and translational initiation region and the transcriptional and translational termination region, so as to be under the regulatory control of the initiation region. This construct will be included in a plasmid, which will include at least one replication system, but may include more than one, where one replication system is employed for cloning during the development of the plasmid and the second replication system is necessary for functioning in the ultimate host. In addition, one or more markers may be present, which have been described previously. Where integration is desired, the plasmid will desirably include a sequence homologous with the host genome.

The transformants can be isolated in accordance with conventional ways, usually employing a selection technique, which allows for selection of the desired organism as against unmodified organisms or transferring organisms, when present. The transformants then can be tested for pesticidal activity.

Suitable host cells, where the pesticide-containing cells will be treated to prolong the activity of the toxin in the cell when the then treated cell is applied to the environment of target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and -positive, include Enterobacteriaceae, such as Escherichia, Erwinia, Shigella, Salmonella, and Proteus; Bacillaceae; Rhizobiceae, such as Rhizobium; Spirillaceae, such as photobacterium, Zymomonas, Serratia, Aeromonas, Vibrio, Desulfovibrio, Spirillum; Lactobacillaceae; Pseudomonadaceae, such as Pseudomonas and Acetobacter; Azotobacteraceae and Nitrobacteraceae. Among eukaryotes are fungi, such as Phycomycetes and Ascomycetes, which includes yeast, such as Saccharomyces and Schizosaccharomyces; and Basidiomycetes yeast, such as Rhodotorula, Aureobasidium, Sporobolomyces, and the like.

Characteristics of particular interest in selecting a host cell for purposes of production include ease of introducing the B.t.i. gene into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as Rhodotorula sp., Aureobasidium sp., Saccharomyces sp., and Sporobolomyces sp.; phylloplane organisms such as Pseudomonas sp., Erwinia sp. and Flavobacterium sp.; or such other organisms as Escherichia, Lactobacillus sp., Bacillus sp., and the like. Specific organisms include Pseudomonas aeruginosa, Pseudomonas fluorescens, Saccharomyces cerevisiae, Bacillus thuringiensis, Escherichia coli, Bacillus subtilis, and the like.

The cell will usually be intact and be substantially in the proliferative form when treated, rather than in a spore form, although in some instances spores may be employed.

Treatment of the microbial cell, e.g., a microbe containing the B.t.i. toxin gene, can be by chemical or physical means, or by a combination of chemical and/or physical means, so long as the technique does not deleteriously affect the properties of the toxin, nor diminish the cellular capability in protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride and cetylpyridinium chloride; alcohols, such as isopropyl and ethanol; various histologic fixatives, such as Bouin's fixative and Helly's fixative (See: Humason, Gretchen L., Animal Tissue Techniques, W.H. Freeman and Company, 1967); or a combination of physical (heat) and chemical agents that preserve and prolong the activity of the toxin produced in the cell when the cell is administered to the host animal. Examples of physical means are short wavelength radiation such as gamma-radiation and X-radiation, freezing, UV irradiation, lyophilization, and the like.

The cells generally will have enhanced structural stability which will enhance resistance to environmental conditions. Where the pesticide is in a proform, the method of inactivation should be selected so as not to inhibit processing of the proform to the mature form of the pesticide by the target pest pathogen. For example, formaldehyde will crosslink proteins and could inhibit processing of the proform of a polypeptide pesticide. The method of inactivation or killing retains at least a substantial portion of the bio-availability or bioactivity of the toxin.

The cellular host containing the B.t.i. insecticidal gene may be grown in any convenient nutrient medium, where the DNA construct provides a selective advantage, providing for a selective medium so that substantially all or all of the cells retain the B.t.i. gene. These cells may then be harvested in accordance with conventional ways. Alternatively, the cells can be treated prior to harvesting.

The B.t.i. cells may be formulated in a variety of ways. They may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about $10^2$ to about $10^4$ cells/mg. These formulations will be administered at about 50 mg (liquid or dry) to 1 kg or more per hectare.

The formulations can be applied to the environment of the dipteran pest(s), e.g., plants, soil or water, by spraying, dusting, sprinkling, or the like.

Following are examples which illustrate procedures, including the best mode, for practicing the invention. These examples should not be construed as limiting. All percentages are by weight and all solvent mixture proportions are by volume unless otherwise noted.

Example 1: Culturing B.t.i. strain HD567

A subculture, or starter culture, of B.t.i. strain HD567 can be used to inoculate to following medium known as LB broth:
Tryptone      10 gm
Yeast extract      5 gm
NaCl      5 gm
5N NaOH      0.6 ml
Water      1000.0 ml

As per standard microbiological techniques, the above medium would be sterilized prior to inoculation and the inoculation would be done using aseptic procedures.

A detailed procedure is as follows:

A series of 150 ml Erlenmeyer flasks containing sterile PWYE medium (peptone 5%, yeast extract 0.1%, NaCl 0.5% in 1 liter of water; adjust pH to 7.5) are inoculated from a petri plate culture of B.t.i. strain HD567. The flasks are incubated at 30°C on a rotary shaker (200 rpm) overnight. From this starter culture, 300 ml of LB broth in a 2-liter flask is inoculated using 7.5 ml of the starter. The LB-broth flasks are incubated under the same conditions as the starter but are harvested after a few hours. The optical density, at 600 nm, is 1.0.

The above procedure can be readily scaled up to large fermentors by procedures well known in the art.

The B.t.i. cells, obtained in the above fermentation, can be isolated by procedures well known in the art. A frequently-used procedure is to subject the harvested fermentation broth to separation techniques, e.g., centrifugation.

Example 2: Cloning of the novel B.t.i. gene

The harvested B.t.i. cells obtained in Example 1 were converted into protoplasts as follows:

The cells were incubated in 10 ml of 10 mM Tris (pH 8.0), 1 mM ethylenediaminetetracetic acid (EDTA), 100mM NaCl, 20% (W/V) sucrose and 50 mg/ml lysozyme for about 2 hr. Then 20 ml of 8% (W/V) sodium dodecyl sulfate (SDS) was added. The mixture was heated to 68°C until it became clear (about 10 min). Next, 3 ml of 5M NaCl was carefully mixed in and the mixture was allowed to stand at 4°C overnight. This was centrifuged at 39,000 xg for 20 min. The supernatant fraction which contained the total cellular DNA was extracted twice with phenol-chloroform, ethanol-precipitated, and purified by cesium chloride density gradient centrifugation using standard methods.

A suitable quantity (100-200 g) of this purified DNA was digested with restriction endonucleases EcoRI and HindIII and then fractionated by electrophoresis on 0.8% agarose gel. The 3.4-3.5 kb region of the gel was selected for DNA recovery. This EcoRI and HindIII fragment of HD567 DNA was electroeluted from the appropriate gel slice and purified. It was then ligated into a sample of the well-known and available plasmid pUC19 which had been cleaved with EcoRI and HindIII and similarly purified by agarose gel electrophoresis. This recombinant ligation mixture was used to transform E. coli strain BB3, available from the Stratagene Cloning Systems, San Diego, CA, to resistance to 100 g/ml ampicillin. This gene bank of about 3.5 kb fragments of HD567 DNA was then screened for the desired gene which codes for a mosquitocidal toxin protein by using a specific hybridization probe.

The hybridization probe was synthesized on an Applied Biosystems, Inc. (Foster City, CA) DNA synthesis machine. The sequence of this probe is as follows:
(5′)CCCACCTGCAGGATCTGTCTTAACCGTACTTAGCGCGGTGCTTCCT(3′)

Once this oligonucleotide was synthesized, it was removed from the synthesis column according to procedures described by Applied Biosystems and purified by polyacrylamide gel electrophoresis (PAGE) according to standard procedures. This pure oligonucleotide was labeled using polynucleotide kinase and [ $\lambda$ - $^{32}$P]ATP, purified and used to detect recombinant clones carrying the desired gene by hybridization to the gene. The hybridizing clone of interest was originally on a plate with very many other colonies and it was purified free of them by picking it, plating at low colony density and repeating the hybridization-screening procedure. The pure clone was used to make a plasmid which was found to have the desired about 3.5 kb EcoRI-HindIII DNA fragment. The recombinant plasmid is designated pBTI3,82-5.

This fragment was sequenced by the standard Sanger dideoxy chain termination method. This sequence has been provided.

Example 3: Subcloning and expression of the B.t.i. toxin gene into Pseudomonas fluorescens

The ca. 3.5 kb DNA fragment carrying the B.t.i. toxin gene is excised from plasmid pBTI3,82-5 with EcoRI and HindIII, purified and subcloned into the EcoRI and HindIII site of the known plasmid pR01614 (NRRL B-12127). Pseudomonas fluorescens is transformed with this plasmid. The expression of B.t.i. toxin by recombinant Pseudomonas cells is verified by its identification on a western blot.

Plasmid pBT13,82-5, containing the B.t.i. toxin gene, can be removed from the transformed host microbe by use of standard well-known procedures. For example, E. coli strain BB3(pBTI3,82-5) can be subjected to cleared lysate isopycnic density gradient procedures, to recover pBTI3,82-5.

A subculture of E. coli strain BB3(pBTI3,82-5) has been deposited in the permanent collection of the Agricultural Research Service Patent Culture Collection (NRRL), Regional Research Center, 1815 North University Street, Peoria, Illinois 61604, USA. The deposit date was 17 September 1987. The accession number is NRRL B-18252.

**Claims**

1. A toxin, active against dipteran insects, having the amino-acid sequence given in the following table, or a mutant thereof having the same protein secondary structure or, if the structure is altered, having substantially the same biological activity:

```
                    5                      10                     15                     20
Met Asn Ser Gly Tyr Pro Leu Ala Asn Asp Leu Gln Gly Ser Met Lys Asn Thr Asn Tyr
ATG AAT TCA GGC TAT CCG TTA GCG AAT GAC TTA CAA GGG TCA ATG AAA AAC ACG AAC TAT


                         25                     30                     35            Pro Ala Ala Ile
Lys Asp Trp Leu Ala Met Cys Glu Asn Asn Gln Gln Tyr Gly Val Asn Pro Ala Ala Ile
AAA GAT TGG CTA GCC ATG TGT GAA AAT AAC CAA CAG TAT GGC GTT AAT CCA GCT GCG ATT


              45                     50                     55                     60
Asn Ser Ser Ser Val Ser Thr Ala Leu Lys Val Ala Gly Ala Ile Leu Lys Phe Val Asn
AAT TCT TCT TCA GTT AGT ACC GCT TTA AAA GTA GCT GGA GCT ATC CTT AAA TTT GTA AAC


              65                     70                     75                     80
Pro Pro Ala Gly Thr Val Leu Thr Val Leu Ser Ala Val Leu Pro Ile Leu Trp Pro Thr
CCA CCT GCA GGT ACT GTC TTA ACC GTA CTT AGC GCG GTG CTT CCT ATT CTT TGG CCG ACT


              85                     90                     95                     100
Asn Thr Pro Thr Pro Glu Arg Val Trp Asn Asp Phe Met Thr Asn Thr Gly Asn Leu Ile
AAT ACT CCA ACG CCT GAA AGA GTT TGG AAT GAT TTC ATG ACC AAT ACA GGG AAT CTT ATT


              105                    110                    115                    120
Asp Gln Thr Val Thr Ala Tyr Val Arg Thr Asp Ala Asn Ala Lys Met Thr Val Val Lys
GAT CAA ACT GTA ACA GCT TAT GTA CGA ACA GAT GCA AAT GCA AAA ATG ACG GTT GTG AAA


              125                    130                    135                    140
Asp Tyr Leu Asp Gln Tyr Thr Thr Lys Phe Asn Thr Trp Lys Arg Glu Pro Asn Asn Gln
GAT TAT TTA GAT CAA TAT ACA ACT AAA TTT AAC ACT TGG AAA AGA GAG CCT AAT AAC CAG


              145                    150                    155                    160
Ser Tyr Arg Thr Ala Val Ile Thr Gln Phe Asn Leu Thr Ser Ala Lys Leu Arg Glu Thr
TCC TAT AGA ACA GCA GTA ATA ACT CAA TTT AAC TTA ACC AGT GCC AAA CTT CGA GAG ACC


              165                    170                    175                    180
Ala Val Tyr Phe Ser Asn Leu Leu Gly Tyr Glu Leu Leu Leu Leu Pro Ile Tyr Ala Gln
GCA GTT TAT TTT AGC AAC TTA CTA GGT TAT GAA TTA TTG TTA TTA CCA ATA TAC GCA CAA


              185                    190                    195                    200
Val Ala Asn Phe Asn Leu Leu Leu Ile Arg Asp Gly Leu Ile Asn Ala Gln Glu Trp Ser
GTA GCA AAT TTC AAT TTA CTT TTA ATA AGA GAT GGC CTC ATA AAT GCA CAA GAA TGG TCT


              205                    210                    215                    220
Leu Ala Arg Ser Ala Gly Asp Gln Leu Tyr Asn Thr Met Val Gln Tyr Thr Lys Glu Tyr
TTA GCA CGT AGT GCT GGT GAC CAA CTA TAT AAC ACT ATG GTG CAG TAC ACT AAA GAA TAT


              225                    230                    235                    240
Ile Ala His Ser Ile Thr Trp Tyr Asn Lys Gly Leu Asp Val Leu Arg Asn Lys Ser Asn
ATT GCA CAT AGC ATT ACA TGG TAT AAT AAA GGT TTA GAT GTA CTT AGA AAT AAA TCT AAT


              245                    250                    255                    260
Gly Gln Trp Ile Thr Phe Asn Asp Tyr Lys Arg Glu Met Thr Ile Gln Val Leu Asp Ile
GGA CAA TGG ATT ACG TTT AAT GAT TAT AAA AGA GAG ATG ACT ATT CAA GTA TTA GAT ATA


              265                    270                    275                    280
Leu Ala Leu Phe Ala Ser Tyr Asp Pro Arg Arg Tyr Pro Ala Asp Lys Ile Asp Asn Thr
CTC GCT CTT TTT GCC AGT TAT GAT CCA CGT CGA TAC CCT GCG GAC AAA ATA GAT AAT ACG
```

```
              285                    290                    295                    300
Lys Leu Ser Lys Thr Glu Phe Thr Arg Glu Ile Tyr Thr Ala Leu Val Glu Ser Pro Ser
AAA CTA TCA AAA ACA GAA TTT ACA AGA GAG ATT TAT ACA GCT TTA GTA GAA TCT CCT TCT


              305                    310                    315                    320
Ser Lys Ser Ile Ala Ala Leu Glu Ala Ala Leu Thr Arg Asp Val His Leu Phe Thr Trp
AGT AAA TCT ATA GCA GCA CTG GAG GCA GCA CTT ACA CGA GAT GTT CAT TTA TTC ACT TGG


              325                    330                    335                    340
Leu Lys Arg Val Asp Phe Trp Thr Asn Thr Ile Tyr Gln Asp Leu Arg Phe Leu Ser Ala
CTA AAG AGA GTA GAT TTC TGG ACC AAT ACT ATA TAT CAA GAT TTA AGA TTT TTA TCT GCC


              345                    350                    355                    360
Asn Lys Ile Gly Phe Ser Tyr Thr Asn Ser Ser Ala Met Gln Glu Ser Gly Ile Tyr Gly
AAT AAA ATT GGG TTT TCA TAT ACA AAT TCT TCT GCA ATG CAA GAA AGT GGA ATT TAT GGA


              365                    370                    375                    380
Ser Ser Gly Phe Gly Ser Asn Leu Thr His Gln Ile Gln Leu Asn Ser Asn Val Tyr Lys
AGT TCT GGT TTT GGT TCA AAT CTT ACT CAT CAA ATT CAA CTT AAT TCT AAT GTT TAT AAA


              385                    390                    395                    400
Thr Ser Ile Thr Asp Thr Ser Ser Pro Ser Asn Arg Val Thr Lys Met Asp Phe Tyr Lys
ACT TCT ATC ACA GAT ACT AGC TCC CCC TCT AAT CGA GTT ACA AAA ATG GAT TTC TAC AAA


              405                    410                    415                    420
Ile Asp Gly Thr Leu Ala Ser Tyr Asn Ser Asn Ile Thr Pro Thr Pro Glu Gly Leu Arg
ATT GAT GGT ACT CTT GCC TCT TAT AAT TCA AAT ATA ACA CCA ACT CCT GAA GGT TTA AGG


              425                    430                    435                    440
Thr Thr Phe Phe Gly Phe Ser Thr Asn Glu Asn Thr Pro Asn Gln Pro Thr Val Asn Asp
ACC ACA TTT TTT GGA TTT TCA ACA AAT GAG AAC ACA CCT AAT CAA CCA ACT GTA AAT GAT


              445                    450                    455                    460
Tyr Thr His Ile Leu Ser Tyr Ile Lys Thr Asp Val Ile Asp Tyr Asn Ser Asn Arg Val
TAT ACG CAT ATT TTA AGC TAT ATA AAA ACT GAT GTT ATA GAT TAT AAC AGT AAC AGG GTT


              465                    470                    475                    480
Ser Phe Ala Trp Thr His Lys Ile Val Asp Pro Asn Asn Gln Ile Tyr Thr Asp Ala Ile
TCA TTT GCT TGG ACA CAT AAG ATT GTT GAC CCT AAT AAT CAA ATA TAC ACA GAT GCT ATC


              485                    490                    495                    500
Thr Gln Val Pro Ala Val Lys Ser Asn Phe Leu Asn Ala Thr Ala Lys Val Ile Lys Gly
ACA CAA GTT CCG GCC GTA AAA TCT AAC TTC TTG AAT GCA ACA GCT AAA GTA ATC AAG GGA


              505                    510                    515                    520
Pro Gly His Thr Gly Gly Asp Leu Val Ala Leu Thr Ser Asn Gly Thr Leu Ser Gly Arg
CCT GGT CAT ACA GGG GGG GAT CTA GTT GCT CTT ACA AGC AAT GGT ACT CTA TCA GGC AGA


              525                    530                    535                    540
Met Glu Ile Gln Cys Lys Thr Ser Ile Phe Asn Asp Pro Thr Arg Ser Tyr Gly Leu Arg
ATG GAG ATT CAA TGT AAA ACA AGT ATT TTT AAT GAT CCT ACA AGA AGT TAC GGA TTA CGC


              545                    550                    555                    560
Ile Arg Tyr Ala Ala Asn Ser Pro Ile Val Leu Asn Val Ser Tyr Val Leu Gln Gly Val
ATA CGT TAT GCT GCA AAT AGT CCA ATT GTA TTG AAT GTA TCA TAT GTA TTA CAA GGA GTT


              565                    570                    575                    580
Ser Arg Gly Thr Thr Ile Ser Thr Glu Ser Thr Phe Ser Arg Pro Asn Asn Ile Ile Pro
TCT AGA GGA ACA ACG ATT AGT ACA GAA TCT ACG TTT TCA AGA CCT AAT AAT ATA ATA CCT
```

```
            585                   590                   595                   600
Thr Asp Leu Lys Tyr Glu Glu Phe Arg Tyr Lys Asp Pro Phe Asp Ala Ile Val Pro Met
ACA GAT TTA AAA TAT GAA GAG TTT AGA TAC AAA GAT CCT TTT GAT GCA ATT GTA CCG ATG


            605                   610                   615                   620
Arg Leu Ser Ser Asn Gln Leu Ile Thr Ile Ala Ile Gln Pro Leu Asn Met Thr Ser Asn
AGA TTA TCT TCT AAT CAA CTG ATA ACT ATA GCT ATT CAA CCA TTA AAC ATG ACT TCA AAT


            625                   630                   635                   640
Asn Gln Val Ile Ile Asp Arg Ile Glu Ile Ile Pro Ile Thr Gln Ser Val Leu Asp Glu
AAT CAA GTG ATT ATT GAC AGA ATC GAA ATT ATT CCA ATC ACT CAA TCT GTA TTA GAT GAG


            645                   650                   655                   660
Thr Glu Asn Gln Asn Leu Glu Ser Glu Arg Glu Val Val Asn Ala Leu Phe Thr Asn Asp
ACA GAG AAC CAA AAT TTA GAA TCA GAA CGA GAA GTT GTG AAT GCA CTG TTT ACA AAT GAC


            665                   670                   675                   680
Ala Lys Asp Ala Leu Asn Ile Gly Thr Thr Asp Tyr Asp Ile Asp Gln Ala Ala Asn Leu
GCG AAA GAT GCA TTA AAC ATT GGA ACG ACA GAT TAT GAC ATA GAT CAA GCC GCA AAT CTT


            685                   690                   695                   700
Val Glu Cys Ile Ser Glu Glu Leu Tyr Pro Lys Glu Lys Met Leu Leu Leu Asp Glu Val
GTG GAA TGT ATT TCT GAA GAA TTA TAT CCA AAA GAA AAA ATG CTG TTA TTA GAT GAA GTT


            705                   710                   715                   720
Lys Asn Ala Lys Gln Leu Ser Gln Ser Arg Asn Val Leu Gln Asn Gly Asp Phe Glu Ser
AAA AAT GCG AAA CAA CTT AGT CAA TCT CGA AAT GTA CTT CAA AAC GGG GAT TTT GAA TCG


            725                   730                   735                   740
Ala Thr Leu Gly Trp Thr Thr Ser Asp Asn Ile Thr Ile Gln Glu Asp Asp Pro Ile Phe
GCT ACG CTT GGT TGG ACA ACA AGT GAT AAT ATC ACA ATT CAA GAA GAT GAT CCT ATT TTT


            745                   750                   755                   760
Lys Gly His Tyr Leu His Met Ser Gly Ala Arg Asp Ile Asp Gly Thr Ile Phe Pro Thr
AAA GGG CAT TAC CTT CAT ATG TCT GGG GCG AGA GAC ATT GAT GGT ACG ATA TTT CCG ACC


            765                   770                   775                   780
Tyr Ile Phe Gln Lys Ile Asp Glu Ser Lys Leu Lys Pro Tyr Thr Arg Tyr Leu Val Arg
TAT ATA TTC CAA AAA ATT GAT GAA TCA AAA TTA AAA CCG TAT ACA CGT TAC CTA GTA AGG


            785                   790                   795                   800
Gly Phe Val Gly Ser Ser Lys Asp Val Glu Leu Val Val Ser Arg Tyr Gly Glu Glu Ile
GGA TTT GTA GGA AGT AGT AAA GAT GTA GAA CTA GTG GTT TCA CGC TAT GGG GAA GAA ATT


            805                   810                   815                   820
Asp Ala Ile Met Asn Val Pro Ala Asp Leu Asn Tyr Leu Tyr Pro Ser Thr Phe Asp Cys
GAT GCC ATC ATG AAT GTT CCA GCT GAT TTA AAC TAT CTG TAT CCT TCT ACC TTT GAT TGT


            825                   830                   835                   840
Glu Gly Ser Asn Arg Cys Glu Thr Ser Ala Val Pro Ala Asn Ile Gly Asn Thr Ser Asp
GAA GGG TCT AAT CGT TGT GAG ACG TCC GCT GTG CCG GCT AAC ATT GGG AAC ACT TCT GAT


            845                   850                   855                   860
Met Leu Tyr Ser Cys Gln Tyr Asp Thr Gly Lys Lys His Val Val Cys Gln Asp Ser His
ATG TTG TAT TCA TGC CAA TAT GAT ACA GGG AAA AAG CAT GTC GTA TGT CAG GAT TCC CAT


            865                   870                   875                   880
Gln Phe Ser Phe Thr Ile Asp Thr Gly Ala Leu Asp Thr Asn Glu Asn Ile Gly Val Trp
CAA TTT AGT TTC ACT ATT GAT ACA GGG GCA TTA GAT ACA AAT GAA AAT ATA GGG GTT TGG
```

9

```
                     885                      890                      895                      900
Val Met Phe Lys Ile Ser Ser Pro Asp Gly Tyr Ala Ser Leu Asp Asn Leu Glu Val Ile
GTC ATG TTT AAA ATA TCT TCT CCA GAT GGA TAC GCA TCA TTA GAT AAT TTA GAA GTA ATT


                     905                      910                      915                      920
Glu Glu Gly Pro Ile Asp Gly Glu Ala Leu Ser Arg Val Lys His Met Glu Lys Lys Trp
GAA GAA GGG CCA ATA GAT GGG GAA GCA CTG TCA CGC GTG AAA CAC ATG GAG AAG AAA TGG


                     925                      930                      935                      940
Asn Asp Gln Met Glu Ala Lys Arg Ser Glu Thr Gln Gln Ala Tyr Asp Val Ala Lys Gln
AAC GAT CAA ATG GAA GCA AAA CGT TCG GAA ACA CAA CAA GCA TAT GAT GTA GCG AAA CAA


                     945                      950                      955                      960
Ala Ile Asp Ala Leu Phe Thr Asn Val Gln Asp Glu Ala Leu Gln Phe Asp Thr Thr Leu
GCC ATT GAT GCT TTA TTC ACA AAT GTA CAA GAT GAG GCT TTA CAG TTT GAT ACG ACA CTC


                     965                      970                      975                      980
Ala Gln Ile Gln Tyr Ala Glu Tyr Leu Val Gln Ser Ile Pro Tyr Val Tyr Asn Asp Trp
GCT CAA ATT CAG TAC GCT GAG TAT TTG GTA CAA TCG ATT CCA TAT GTG TAC AAT GAT TGG


                     985                      990                      995                      1000
Leu Ser Asp Val Pro Gly Met Asn Tyr Asp Ile Tyr Val Glu Leu Asp Ala Arg Val Ala
TTG TCA GAT GTT CCA GGT ATG AAT TAT GAT ATC TAT GTA GAG TTG GAT GCA CGA GTG GCA


                     1005                     1010                     1015                     1020
Gln Ala Arg Tyr Leu Tyr Asp Thr Arg Asn Ile Ile Lys Asn Gly Asp Phe Thr Gln Gly
CAA GCG CGT TAT TTG TAT GAT ACA AGA AAT ATT ATT AAA AAT GGT GAT TTT ACA CAA GGG


                     1025                     1030                     1035                     1040
Val Met Gly Trp His Val Thr Gly Asn Ala Asp Val Gln Gln Ile Asp Gly Val Ser Val
GTA ATG GGG TGG CAT GTA ACT GGA AAT GCA GAC GTA CAA CAA ATA GAT GGT GTT TCT GTA


                     1045                     1050                     1055                     1060
Leu Val Leu Ser Asn Trp Ser Ala Gly Val Ser Gln Asn Val His Leu Gln His Asn His
TTG GTT CTA TCT AAT TGG AGT GCT GGC GTA TCT CAA AAT GTC CAT CTC CAA CAT AAT CAT


                     1065                     1070                     1075                     1080
Gly Tyr Val Leu Arg Val Ile Ala Lys Lys Glu Gly Pro Gly Asn Gly Tyr Val Thr Leu
GGG TAT GTC TTA CGT GTT ATT GCC AAA AAA GAA GGA CCT GGA AAT GGG TAT GTC ACG CTT


                     1085                     1090                     1095                     1100
Met Asp Cys Glu Glu Asn Gln Glu Lys Leu Thr Phe Thr Ser Cys Glu Glu Gly Tyr Ile
ATG GAT TGT GAG GAG AAT CAA GAA AAA TTG ACG TTT ACG TCT TGT GAA GAA GGA TAT ATT


                     1105                     1110                     1115                     1120
Thr Lys Thr Val Asp Val Phe Pro Asp Thr Asp Arg Val Arg Ile Glu Ile Gly Glu Thr
ACG AAG ACA GTA GAT GTA TTC CCA GAT ACA GAT CGT GTA CGA ATT GAG ATA GGC GAA ACC


                     1125                     1130                     1135
Glu Gly Ser Phe Tyr Ile Glu Ser Ile Glu Leu Ile Cys Met Asn Glu ***
GAA GGT TCG TTT TAT ATC GAA AGC ATT GAA TTA ATT TGC ATG AAC GAG TGA TTA ATA AAA



AAT ACC TAA AGC TTA TTA AAC ACT GGA GAA AGT TTT CTC CAT GGT TTT TAA TTT CTG CAT



TTA TTA ATT TCT GGT ACA AAA AAT ATA TAG AAA ACA TAA AAA ATA GAT ATC TAG A
```

2. A DNA molecule including the nucleotide sequence tabulated in claim 1 or a sequence encoding the amino-acid sequence tabulated in claim 1.

3. A prokaryotic or eukaryotic host into which DNA according to claim 2, as a DNA transfer vector, has been transferred and replicated.

4. A bacterial host capable of expressing a toxin having the amino-acid sequence tabulated in claim 1.

5. Excherichia coli strain BB3(pBTI3,82-5), NRRL B-18252.

6. A live microorganism comprising a gene coding for at least a fragment of the toxin having the amino-acid sequence tabulated in claim 1, the toxin or fragment being biocidal for a dipteran insect, and the toxin or fragment being maintained at a level such that, upon ingestion of the microorganism by the insect, a growth-inhibiting of the toxin or fragment can be ingested.

7. A microorganism according to claim 6, which is a species of Pseudomonas (preferred), Azotobacter, Erwinia, Serratia, Klebsiella, Rhizobium, Rhodopseudomonas, Methylophilius, Agrobacterium, Acetobacter or Alcaligenes; which is preferably pigmented and phylloplane-adherent.

8. A method for controlling dipteran insects, which comprises administering to the insects or to their environment a microorganism according to claim 6 or claim 7.

9. A method according to claim 8, wherein the administration is to the rhizosphere, to the phylloplane, or to a body of water.

10. Treated, substantially intact unicellular microorganism cells containing an intracellular toxin which is the result of expression of a Bacillus thuringiensis toxin gene which codes for a toxin having the amino-acid sequence tabulated in claim 1, wherein treatment prolongs the insecticidal activity when the cells are applied to the environment of a target insect.

11. Cells according to claim 10, which are prokaryote cells selected from Enterobacteriaceae, Bacillaceae, Rhizobiaceae, Spirillaceae, Lactobacillaceae, Pseudomonadaceae (preferred), Azotobacteraceae and Nitrobacteraceae, or lower eukaryote cells selected from Phycomycetes, Ascomycetes and Basidiomycetes.

12. Cells according to claim 10, which are of a pigmented bacterium, yeast, or fungus.

13. Cells according to any of claims 10 to 12, which are treated with iodine.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 195 285 (UNIVERSITY OF GEORGIA RESEARCH FOUNDATION) * Claims 1,2,12,13,18,19,23,30 * | 1-5 | C 12 N 15/00 C 12 N 1/20 C 12 P 21/02 // (C 12 N 1/20 C 12 R 1:19 ) |
| Y | | 6-13 | |
| X | EP-A-0 153 166 (SYNTRO CORP.) * Claims 1,2,5,6; page 9, line 14 - page 15e * | 1-5 | |
| Y | | 6-13 | |
| X | NUCLEIC ACIDS RESEARCH, vol. 15, no. 17, 11th September 1987, page 7195, IRL Press Limited, Eynsham, Oxford, GB; E.S.WARD et al.: "Nucleotide sequence of a Bacillus thuringiensis var. isaelensis gene encoding a 130 kDa delta-endotoxin" * The whole article * | 1 | |
| Y | EP-A-0 185 005 (MONSANTO) * Claims * | 6-9 | TECHNICAL FIELDS SEARCHED (Int. Cl.4) |
| Y | EP-A-0 200 344 (MYCOGEN CORP.) * Page 3, column 4, line 21; claims * | 6-9 | C 12 N C 12 P C 12 R |
| Y | EP-A-0 192 319 (MYCOGEN) * Page 5, line 24; page 6, line 25 - page 9, line 2; page 12, line 10 - page 14, line 15; example 1; claims 1-3,6,8 * | 10-13 | |

-/-

The present search report has been drawn up for all claims

| Place of search | Date of completion of the search | Examiner |
|---|---|---|
| THE HAGUE | 09-11-1988 | PULAZZINI A.F.R. |

European Patent Office

**EUROPEAN SEARCH REPORT**

Application Number

EP 88 30 8498

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | FEMS MICROBIOLOGY LETTERS, vol. 30, 1986, Published by Elsevier, pages 211-214, Federation of European Microbiological Societies; B.VISSER et al.: "The mosquitocidal activity of Bacillus thuringiensis var. israelensis is associated with Mr 230 000 and 130 000 crystal proteins" | | |
| A | JOURNAL OF BACTERIOLOGY, vol. 166, no. 3, June 1986, pages 801-811, American Society for Microbiology; L.THORNE et al.: "Structural similarity between the Lepidoptera- and Diptera-Specific insecticidal endotoxin genes of Bacillus thuringiensis subsp. "kurstaki" and "israelensis""" | | |
| P,X | NUCLEIC ACIDS RESEARCH, vol. 16, no. 4, 25th February 1988, pages 1637-1638, IRL Press Limited, Oxford, GB; S.TUNGPRADUBKUL et al.: "The complete nucleotide sequence of a 130 kDa mosquito-larvicidal delta-endotoxin gene of Bacillus thuringiensis var. israelensis" * The whole article * | 1 | TECHNICAL FIELDS SEARCHED (Int. Cl.4) |
| P,X | EUR. J. BIOCHEM., vol. 173, no. 1, 5th April 1988, pages 9-16, FEBS; W.CHUNGJATUPORNCHAI et al.: "Common features of Bacillus thuringiensis toxins specific for diptera and lepidoptera" * Page 12, figure 4 * | 1-4 | |

The present search report has been drawn up for all claims

| Place of search | Date of completion of the search | Examiner |
|---|---|---|
| THE HAGUE | 09-11-1988 | PULAZZINI A.F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)